Europäisches Patentamt
European Patent Office
Office européen des brevets

(19)

(11) Publication number: **0 448 185 A2**

(12) # EUROPEAN PATENT APPLICATION

(21) Application number: 91201460.2

(22) Date of filing: 14.07.88

(51) Int. Cl.[5]: A61K 7/09

This application was filed on 13.06.1991 as a divisional application to the application mentioned under INID code 60.

(30) Priority: 16.07.87 US 74206
16.07.87 US 74207
16.07.87 US 74208

(43) Date of publication of application: 25.09.91 Bulletin 91/39

(60) Publication number of the earlier application in accordance with Art.76 EPC: 0 299 764

(84) Designated Contracting States: AT BE CH DE ES FR GB GR IT LI NL SE

(71) Applicant: UNILEVER PLC
Unilever House Blackfriars
London EC4P 4BQ(GB)
(84) GB

Applicant: UNILEVER NV
Burgemeester s'Jacobplein 1 P.O. Box 760
NL-3000 DK Rotterdam(NL)
(84) BE CH DE ES FR GR IT LI NL SE AT

(72) Inventor: Suita-Mangano, Patricia
1 Five Oaks Lane
Valley Cottage, New York 10989(US)

(74) Representative: Ford, Michael Frederick et al
MEWBURN ELLIS 2 Cursitor Street
London EC4A 1BQ(GB)

(54) **Hair treatment composition.**

(57) Shaping of keratinous material, such as permanent waving of human hair, is carried out using disulfide compounds to effect reoxidation.

Preferred disulfide compounds are cystamine

$NH_2-CH_2CH_2-S - S - CH_2CH_2-NH_2$

and glutathione disulphide

EP 0 448 185 A2

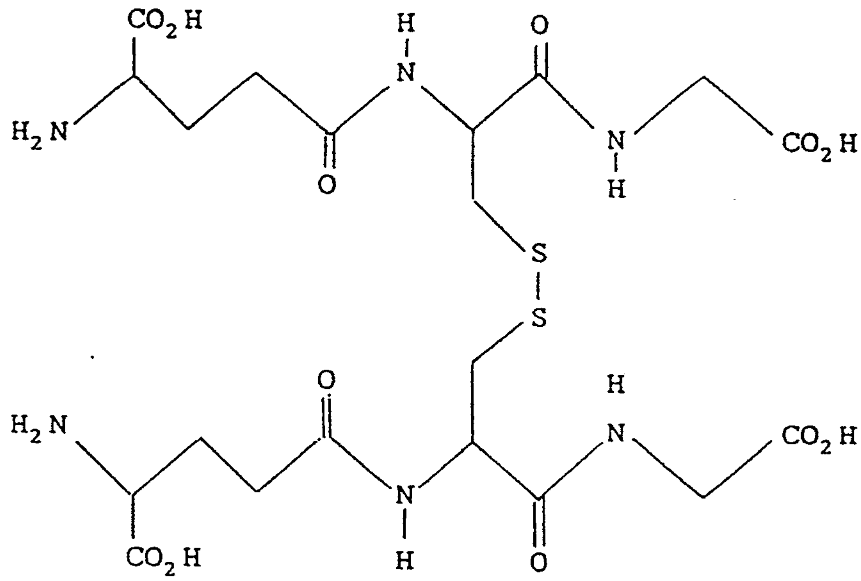

The present invention relates to the treatment of keratinous materials to effect permanent waving or straightening.

Keratinous material, which occurs in animal hair, such as camel hair, mohair, wool, horsehair, cattle hair, fur and the like, and feathers, such as from poultry and, in particular, human hair consists of long polypeptide chains crosslinked to one another by means of occasional disulfide linkages. The disulfide linkages act to hold the hair in a permanent shape or configuration. Disruption of the disulfide linkages permits the polypeptide chains to function independently, allowing for the deformation of the shape of the hair without elasticity. Rupture of disulfide linkages may be accomplished by the use of various reducing agents, such as inorganic sulfides, sulfites, hydrosulfites and cyanides, mercaptans, thioglycolic acids, and various other compounds. Sulfhydryl groups are formed in place of the disulfide linkages.

In the cold-waving of hair, reduction of the disulfide linkages in hair is commonly performed in order to produce a permanent set, as in permanent waving, curling, or de-kinking of hair. The shaping of hair has conventionally been carried out by contacting the hair with a reducing agent in the form of liquids, creams, or gels while the hair has been mechanically formed into the desired new shape. The reducing composition is applied to the hair for a sufficient time to allow shaping to cccur by the reductive disruption of the disulfide linkages. The reducing composition is then washed from the hair, and the normal resilience of the hair may then be regained by restoring the crosslinkages either by means of oxidizing agents or by treatment with the various crosslinking agents.

If permanent straightening of the hair is desired, the reducing lotion is generally applied in a thickened form such as, for instance, in a cream, and evenly distributed throughout the hair by combing. The hair is combed more or less continuously and maintained in a straightened condition for a period of time sufficient to allow rupture of the disulfide linkages. The shaping agent is then washed out with water and the set is then fixed as with an oxidizing agent or a crosslinking agent.

The restoration of the disulfide linkages is important in order to increase tensile strength, as well as remove the sulfhydryl groups as reactive sites. The desired crosslinking may be obtained by oxidation, as by heating in the presence of hair or by reacting with hydrogen peroxide solution. Bromates are frequently employed as oxidizing agents in the setting of permanently waved hair. Valuable properties may also be secured by the use of crosslinking agents, such as alkylene dihalides or dihalocarboxylic acids (US Patent No. 2,739,033) or dimaleimides (US Patent No. 2,850,351). By the use of crosslinking agents, it is possible to convert sulfhydryl linkages to cross linkages.

We have now found that it is possible to improve the known process of restoration by disulfide linkages by use of compounds which themselves incorporate disulfide linkages, utilising these in the step of restoration disulfide linkages.

According to a first aspect of this invention, there is provided a process for treating keratinous material in which disulfide linkages have been ruptured to form pendant sulfhydryl groups by the action of a reducing agent, which process comprises contacting said keratinous material with a compound which contains a disulfide linkage and is effective, preferably when applied in aqueous solution at Ph 6 to 9, to reoxidise said pendant sulfhydryl groups to disulfide linkages with concomitant rupture of its own disulfide linkage.

The invention also includes compositions including these disulfide compounds.

While a wide group of keratinous fiber can be treated in accordance with the present invention, including animal hair such as camel hair, mohair, wool, horsehair, cattle hair, hog bristles and the like; and feathers, such as from chicken, duck, turkey and the like, the invention is particularly directed to waving human hair whether in vivo or in vitro, i.e. in the form of wigs. The invention is particularly directed to "cold waving" and hair straightening systems. The invention will be discussed and preferred forms described in connection with the treatment of human hair to effect deformation to a desired shape.

The step of rupturing disulfide linkages to form pendant sulfhydryl groups may be carried out using a sulfite/bisulfite reducing agent and this may be used jointly with a reduction improver which is a sulfhydryl compound. The use of certain sulfhydryl compounds to enhance the action of a sulfite/bisulfite reducing system is the subject of our copending European application from which the present application is divided.

The term "sulfite/bisulfite" is intended to mean sulfite and/or bisulfite in its disulfide reducing forms including alkali metal and ammonium salts.

The sulfites and/or bisulfites which can be used in the invention are those normally used in hair waving such as the sodium and ammonium salts. The sulfites and/or bisulfites can be used at neutral or even slightly acidic pH. The amount of the reducing agent used should normally be sufficient to rupture a sufficient proportion of the disulfide linkages for effective hair waving or hair straightening as would be appreciated by one of ordinary skill in the art.

The sulfite/bisulfite may be used at a concentration conventionally used in hair waving and straightening

compositions to effect that end. In hair waving, a broad range of from about 4% to about 20% and preferably from about 4% to 10% can be used. For hair straightening, a similar broad range can be used and the preferred range is from about 5% to about 12%. These percentages are by weight of the anion based on the weight of the waving lotion. The amount used depends on the condition of the hair, lower amounts being preferred for highly bleached hair.

A reduction-enhancing, water soluble sulfhydryl compound is preferably employed with the sulfite-bisulfite in an aqueous solution. It may be of general formula

Z - R - SH

in which Z is a water-solubilising functional group and R is a divalent moiety containing at least two carbon atoms.

Preferred reduction improvers are aminothiols of the formula $NH2C_nH_{2n}SH$ wherein n is an integer of from 2 to about 10, preferably 2. This group of compounds can be illustrated by the preferred compound cysteamine, also known as 2-aminoethanethiol, betamercaptoethylamine or 2-aminoethyl mercaptan.

The amino thiol is used in an amount sufficient to improve the waving effects (reduction effects) of the sulfite/bisulfite waving system. The amounts can vary depending on the formulation of the waving lotion and the effectiveness of the reduction improvers. The amount of amino thiol used can also vary depending on the type and condition of hair. Generally, the sulfhydryl compound is used in an add-on weight percentage per volume of the waving lotion ranging from about 0.1% to about 10%.

The modified reducing system incorporating a sulfhydryl compound can be used to replace the sulfite/bisulfite reducing system in commercial formulations. Use times, temperatures, pH and other such conditions will generally be the same or similar to those presently in use for such commercial formulations. These can be easily determined by one of ordinary skill in the art. Sulfite/bisulfite systems of neutral to slightly alkaline are preferred, e.g. pH 6 to 9.

By the breaking of the disulfide bonds to form free sulfhydryl groups pendant on the hair, the hair can be formed or shaped as desired such as by winding on rollers or pins, or combed out as in the case of hair straightening. The breaking of the disulfide bonds is generally accomplished in accordance with the usual practice, which involves applying the reducing agent to the hair wound on curlers. Heat can be provided at this point.

Alternatively the process of this invention may be preceded by a conventional reduction step to rupture the disulfide linkages.

The reducing agents most commonly used in cold waving hair lotions for rupturing cystine linkages are thiols or mercaptans as well as sulfites and/or bisulfites. A number of mercaptans can only provide acceptable efficiency at high pH whereas others with a lower pK and a high ionization constant can be effective at lower pH levels. For example, the ammonium salt of thioglycolic acid can provide acceptable waving efficiency (reduction) if the pH of the solution exceeds 9. Other compounds such as thioglycolamides or glycol thioglycolates, sulfites and/or bisulfites can be used at neutral or even slightly acidic pH. The following are mercaptans and thiols which have commonly been used in cold waving lotions: thioglycolic acid, thiolactic acid, cysteine, thioglycerol, thioglycolic hydrazide, thioglycolamide, glycerol monothioglycolate, beta-mercapto-propionic acid, N-hydroxyethyl mercapto-acetamide, N-methyl mercapto-acetamide, beta-mercapto-ethylamine, beta-mercapto-propionamide, 2-mercapto-ethanesulfonic acid, dimercapto-adipic acid, dithiothreitol, homocysteinethiolactone, cysteine derivatives, and polythiol derivatives formed by the addition of cysteamine onto a maleic anhydride-alkylvinylether copolymer.

The amount of the reducing agent used is that sufficient to rupture a sufficient number of disulfide bonds for effective hair waving or hair straightening as would be appreciated by one of ordinary skill in the art. As mentioned above, the usual practice involves applying the reducing agent to the hair wound on curlers. Heat can be provided at this point.

The disulfide compound used to restore disulfide linkages in the present invention may be of the general formula

Z - R - S - S - R - Z

where Z is a water-solubilising group, notably amino and R is a divalent moiety of at least two carbon atoms.

One preferred category of compounds is of the formula

$NH_2$ - R - S - S - R - $NH_2$

where R is alkylene of 2 to 10 carbon atoms. Especially preferred in this category is cystamine

$NH_2 - CH_2CH_2 - S - S - CH_2CH_2 - NH_2$

Another preferred compound is glutathione disulfide whose formula is

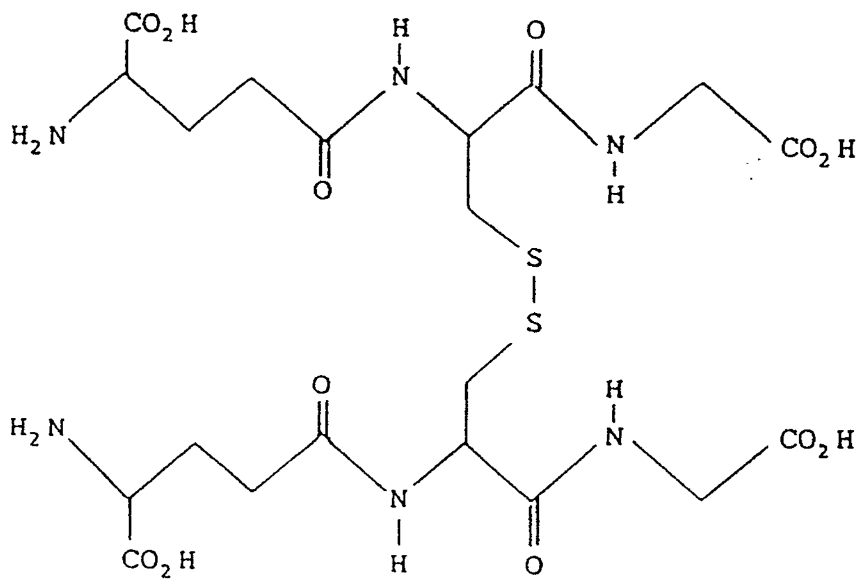

To carry out the invention, the deformed hair, while curled or straightened, is wetted with a crosslinking composition containing the disulfide compound of the invention, suitably in water or in a carrier or base such as a lotion or cream, preferably buffered. The compound is preferably water soluble. The carriers may be of known types, similar to those presently in use in waving and "neutralizing" compositions. The water or carrier desirably holds the crosslinking reagent in contact with the hair for a period of time sufficient to effect permanent setting of the hair. The disulfide compound is applied under conditions conducive to effecting crosslinking. A pH of between about 5 and about 9 (less than that which would cause permanent breakdown of the hair protein) has been found effective for that purpose.

The disulfide compound is preferably applied to the hair in a buffered system, and particularly a buffered system designed to maintain the pH between about 6 and about 9, and preferably between about 7 and 8. Any of a combination of alkali metal phosphates, acetates, borates and the like which are non-reactive with the crosslinking reagent to thus destroy crosslinking sites, can be used to maintain the pH of the hair treated with the crosslikking composition within the range specified. Any pH effects caused by the reducing agent can be offset by thorough washing of the hair with water prior to the application of the crosslinking composition.

The crosslinking composition can also contain a wetting agent or surfactant which is non-reactive with the crosslinking reagent or the hair to destroy crosslinking sites. The surfactant can be anionic, such as soaps, and alkyl sulfates, such as sodium dodecyl sulfate; cationic such as quaternary ammonium compounds; nonionic, such as glycol esters, glycerol esters, sorbitan esters, polyoxyalkalene esters, polyoxyalkalene ethers, and modified lanolin, as well as amphoteric surfactants. The surfactant is used in an amount sufficient to assist in wetting the hair with the crosslinking reagent. The amount depending on the efficiency of the surfactant.

The disulfide compounds can be applied to the hair alone after reduction or in combination with a reducing agent that is compatible with the disulfide compounds. The use of the single-step system allows for the reduction and immediate crosslinking of the sulfhydryl groups.

The hair is treated for a period of time sufficient to effect the crosslinking to provide the curl and, in some instances, tensile strength increases desired. Illustrative times include from about 3 minutes to as long as may be desired, provided that a long time does not cause deterioration of the hair. Lesser times can be used if lesser effect on the hair is desired.

The disulfide compound is preferably applied in aqueous solution at a temperature between the ranges of about 10° C (50° F) and 93° C (200° F). Time of treatment may vary within wide limits depending on the temperature of the solution, the particular reducing and crosslinking agents used, and the nature of the keratinous material being treated.

The hair can be further treated with presently used neutralizing agents and crosslinking reagents in order to oxidize any free SH groups to disulfide linkages as would be appreciated by one of ordinary skill in the art. The oxidizing or neutralizing chemicals used can be any of the oxidizing agents capable of restoring the disulfide linkages in the hair keratin during the resetting stage, such as aqueous solution of hydrogen peroxide, alkali metal bromates, alkali metal perborates, urea hydrogen peroxide, sodium sesquicarbonate, etc. Rinsing alone with water may restore the broken linkages as well, but it will be much slower.

The permanent waving systems of the invention can be designed for professional and home application. The system and its compositions can contain ingredients normal to such compositions. Fragrance compounds, coloring agents, thickening agents, opacifying agents, sequestering agents, solubilizing agents, gelling agents, conditioning agents, etc., may be added to compositions of this invention in amount conventionally used in hair waving and straightening compositions. Any compound which will react with the sulfhydryl or as the case may be disulfide compounds of this invention to remove or neutralize them is desirably avoided. Conventional ingredients are fully outlined in The Science of Hair Care, edited by Charles Zviak, Vol. 7 of a series entitled Dermatology, Marcel Dekker, Inc. 1986, which is incorporated herein by reference.

As used herein, the term "% weight per volume" is intended to mean "grams per 100 millilitres".

The present invention is more fully illustrated in the Examples which follow. Example 1 illustrates reduction using sulfite/bisulfite and the sulfhydryl compound cysteamine. Examples 2 to 5 illustrate restoration of disulfide linkages in accordance with the invention.

All neutralization (reoxidation), unless otherwise stated, was performed using the neutralizer provided with the commercial waving lotion used in the Examples for 5 minutes. All rinsing was performed with running water for 2 minutes. All times are in minutes.

EXAMPLE 1

Swatches (0.5 grams; 20.3 centimeters in length) of European brown Caucasian hair wrapped around curling rods with a center diameter of 1.27 centimeters were treated with a commercially available sulfite/bisulfite waving lotion containing 2.5% cysteamine for 45 minutes to rupture the disulfide bonds. Control swatches were treated in like manner with the commercial sulfite/bisulfite waving lotion without cysteamine.

The commercial waving lotion used in the Example was of the following formulation:

| Hair Waving Lotion | Parts by Weight |
|---|---|
| Water, deionised | 73.89 |
| Polyoxethylene (23) lauryl ether | 1.00 |
| Disodium EDTA | 0.05 |
| Quaternary copolymer of vinyl pyrrolidone/ dimethylaminoethyl methacrylate | 0.60 |
| PEG-75 lanolin | 0.25 |
| Ammonium bisulfite | 6.50 |
| Ammonium sulfite | 1.20 |
| Ammonia to pH 7.00 | 0.51 |
| Urea | 16.00 |
| | 100.00 |

These swatches were then rinsed for 2 minutes with running tap water, neutralised using the neutralizer provided with the commercial hair waving lotion for 5 minutes followed by a 2 minute water rinse.

Hair swatches cut to 17.8 centimeters were analyzed for hanging curl length immediately after unrolling the hair swatches, after 2 hours of gentle drying, after 1 hour of soaking in 4 liters of tap water containing a few drops of 29% sodium dodecyl sulfate, and after another 2 hours of drying. The lengths were compared during the fifth hour, i.e., the final drying state. Curl lengths better than that obtained using the complete commercial hair waving system as control were seen with the addition of cysteamine.

The following results, which are the average of two experiments, were obtained. A difference of 0.5 centimeter is considered a significant difference.

TABLE 1

| | Curl Length (Centimeters) | | | |
|---|---|---|---|---|
| | Wet 0 hr | Dry 2 hr | Solution 1 hr | Dry 2 hr |
| Commercial waving lotion Without cysteamine (control) | 16.3 | 15.7 | 2.5 | 16.8 |
| With cysteamine | 12.7 | 15.0 | 4.8 | 16.0 |

Temperature 19.4°C (67°F), Relative Humidity 38%

The swatches treated in accordance with the invention gave improved curl tightness vis-a-vis the commercial lotion, and were analyzed for luster, silky feel and combability. Appearance results comparable with that obtained with the commercial lotion-treated hair were obtained.

EXAMPLE 2

Swatches (0.5 grams; 20.3 centimeters in length) of European brown Caucasian hair wrapped around curling rods with a central diameter of 1.27 centimeters were treated with a commercially available waving lotion (Clairol® Professional Kind to Hair Perm System) for 20 minutes to rupture the disulfide bonds. These swatches were then rinsed with running tap water. Some swatches were treated with 10 milliliters of a crosslinking solution containing about 2.5% weight per volume of either cystamine or glutathione disulfide, in each case in 200 millimolar sodium phosphate buffer (pH 8.5) with 1 millimolar EDTA at a final pH of about 7 for 20 minutes. The tresses were rinsed again under running tap water. The Clairol® Perm System control was neutralized using the neutralizer provided with the commercial hair waving system followed by a water rinse. The buffer treatment without either disulfide and without neutralization was conducted for 20 minutes.

Hair swatches cut to 17.8 centimeters were analyzed for hanging curl length immediately after unrolling the hair swatches, after 2 hours of gentle drying, after 1 hour of soaking in 4 liters of tap water containing a few drops of 29% sodium dodecyl sulfate, and after another 2 hours of drying. The lengths were compared during the fifth hour, i.e., the final drying state. Curl lengths comparable to that obtained using the commercial hair waving system with neutralization (Clairol® Perm System) were seen when either disulfide was used without neutralization. Hair swatches treated with buffer alone, i.e., no crosslinking disulfide, for 20 minutes and no neutralization gave very little curl.

The following results, which are the average of two experiments, were obtained. The data are reported in centimeters of hanging hair, the data for solution being measurements made while the curl was in the solution. A reduction in length is an indication of the degree of curl.

TABLE 2

| | Curl Length (Centimeters) | | | |
|---|---|---|---|---|
| | Wet 0 hr | Dry 2 hr | Solution 1 hr | Dry 2 hr |
| Control Commercial Waving System With Neutralization | 13.0 | 13.7 | 3.8 | 15.7 |
| Cystamine Without Neutralization | 10.4 | 13.0 | 2.5 | 15.7 |
| Glutathione Disulfide Without Neutralization | 11.7 | 13.4 | 7.9 | 15.7 |
| Buffer Without Neutralization | 13.7 | 13.7 | 5.0 | 16.5 |

The above treated swatches were analyzed for luster, silky feel and combability. Both disulfide compounds gave appearance results comparable with or better than the commercial lotion-treated hair or untreated hair, e.g., better looking curls, more luster, and more silkiness than the commercially treated hair swatches.

EXAMPLE 3

Example 2 was repeated with the following changes. Cystamine or glutathione disulfide was used in an amount of 5% weight per volume in 200 millimolar phosphate buffer containing 1 millimolar EDTA, final pH 6.8. Some samples were treated with one or other disulfide and rinsed as in Example 2. Further samples were treated with disulfide for 12 minutes, rinsed with water, then neutralized using the neutralizer from the commercial waving system and rinsed with water. The buffer treatment after disulfide bond severing was conducted for 12 minutes, then rinsed and one sample was subsequently neutralized with the commercial neutralizer followed by a water rinse. The following results were obtained:

TABLE 3

| | Curl Length (Centimeters) Wet 0 hr | Dry 2 hr | Solution 1 hr | Dry 2 hr |
|---|---|---|---|---|
| Control | | | | |
| Commercial Waving System With Neutralization | 13.2 | 14.7 | 2.5 | 15.7 |
| Cystamine | | | | |
| Without Neutralization | 12.7 | 14.5 | 11.2 | 15.7 |
| With Neutralization | 12.4 | 14.2 | 3.0 | 15.2 |
| Glutathione Disulfide | | | | |
| Without Neutralization | 12.7 | 14.5 | 9.4 | 16.3 |
| With Neutralization | 13.2 | 14.5 | 3.0 | 15.7 |
| Buffer | | | | |
| Without Neutralization | 16.3 | 16.0 | 8.6 | 16.8 |
| With Neutralization | 13.7 | 14.7 | 3.8 | 15.7 |

EXAMPLE 4

The procedure of Example 2 was repeated using 10 milliliters per curl of a glutathione disulfide crosslinking solution having 2.5% weight per volume glutathione disulfide in 200 millimolar sodium phosphate buffer (pH 8.5) and 1 millimolar EDTA. The following results were obtained:

TABLE 4

| | Curl Length (Centimeters) Wet 0 hr | Dry 2 hr | Solution 1 hr | Dry 2 hr |
|---|---|---|---|---|
| Control | | | | |
| Commercial Waving System With Neutralization | 13.7 | 14.7 | 3.0 | 15.7 |
| Glutathione Disulfide | | | | |
| Without Neutralization | 14.5 | 14.7 | 14.0 | 16.3 |
| With Neutralization* | 12.7 | 14.5 | 4.3 | 15.5 |
| Buffer | | | | |
| Without Neutralization | 14.5 | 15.5 | 5.6 | 16.5 |
| With Neutralization* | 12.4 | 14.5 | 3.8 | 15.2 |

Temperature, 18°C, Relative Humidity 32%

*Neutralization with neutralizer supplied with commercial waving system with water rinse.

EXAMPLE 5

Swatches of hair (as defined in Example 2) were treated on a curler. For control, a hair sample was contacted for 20 minutes with a commercial waving lotion (Clairol® Perm System) to rupture disulfide bonds, rinsed in flowing tap water, neutralized, i.e., reoxidized, utilizing the neutralizer accompanying the commercial waving system, and rinsed. Second and third groups of hair were treated with a commercial waving lotion (Clairol® Perm System) for 20 minutes to rupture disulfide bonds, rinsed in flowing tap water, treated with 10 milliliters of a crosslinking solution of either cystamine or glutathione disulfide in an amount of 2.5% weight per volume in 200 millimolar sodium phosphate buffer and 1 millimolar EDTA (pH 8.5) for 20 minutes (final pH 7.0 to 7.5), rinsed in running tap water, neutralized, and rinsed. A fourth hair sample was treated the same as in the preceding procedure, except that there was no disulfide compound in the buffer.

The following results were obtained:

TABLE 3

| | Curl Length (Centimeters) | | | |
|---|---|---|---|---|
| | Wet 0 hr | Dry 2 hr | Solution 1 hr | Dry 2 hr |
| Control Commercial Waving System, Neutralized | 13.3 | 14.0 | 2.5 | 14.6 |
| Cystamine, Neutralized | 12.7 | 14.0 | 2.5 | 14.6 |
| Glutathione Disulfide, Neutralized | 12.1 | 14.0 | 3.6 | 14.6 |
| Buffer, Neutralized | 12.7 | 14.0 | 3.2 | 14.6 |

## Claims

1. A process for treating keratinous material in which disulfide linkages have been ruptured to form pendant sulfhydryl groups by the action of a reducing agent which comprises contacting said keratinous material with a compound which contains a disulfide linkage and is effective to reoxidize said pendant sulfhydryl groups to disulphide linkages with concomitant rupture of its own disulfide linkage.

2. A process according to claim 1 wherein the disulfide compound has the general formula

   Z - R - S - S - R - Z

   wherein Z is a functional group including -OH or -NH and R is a divalent moiety including at least two carbon atoms.

3. A process according to claim 2 wherein the disulfide compound has the general formula

   $H_2N - R - S - S - R - NH_2$

   wherein R is alkylene of 2 to 10 carbon atoms.

4. A process according to claim 3 wherein the disulfide compound is cystamine.

5. A process according to claim 2 wherein the disulfide compound is glutathione disulfide.

6. A process according to any one of the preceding claims wherein the disulfide compound is applied in aqueous solution at a pH in the range from 6 to 9.

7. A process according to any one of the preceding claims wherein the keratinous material is human hair.

8. A process according to any one of the preceding claims preceded by contacting the keratinous material with a sulfite/bisulfite reducing agent for the disulfide linkages in keratin in combination with a sulfhydryl compound as a reduction improver.

9. A process according to claim 8 wherein the sulfhydryl compound has the general formula

Z - R - SH

wherein Z is a water solubilizing group and R is a divalent moiety including at least two carbon atoms.

10. A process according to claim 9 wherein the sufhydryl compound has the general formula

$H_2N$ - R - SH

wherein R is alkylene of 2 to 10 carbon atoms, and may bear substituent groups.

11. A process according to claim 10 wherein the sulfhydryl compound is cysteamine.

12. A process according to claim 11 wherein the sulfhydryl compound is applied in aqueous solution with the sulfite/bisulfite at a pH in the range from 6 to 9.